# EUROPEAN PATENT APPLICATION

(11) **EP 2 328 126 A1**
(43) Date of publication of application: **01.06.2011**
(21) Application number: 09177356.4
(22) Date of filing: 27.11.2009
(51) Int. Cl.: G06T 7/00, G06T 11/00, G06F 19/00

(54) **Genome-wide association study identifying determinants of facial characteristics for facial image generation**

(71) Applicant: Technical University of Denmark, 2800 Lyngby (DK)
(72) Inventor: Nielsen, Henrik Bjørn, 2800, Lyngby (DK); Jarmer, Hanne Østergaard, 2800, Lyngby (DK)
(74) Representative: Høiberg A/S

(57) **Abstract**

The present invention relates to a method for the generation of a facial composite from the genetic profile of a DNA-donor. The method comprises the steps of a) subjecting a biological sample to genotyping thereby generating a profile of genetic markers associated to numerical facial descriptors (NFD) for said sample, b) reverse engineer a NFD from the profile of the associated genetic variants and constructing a facial composite from the reverse engineered numerical facial descriptors (NFDs). The present invention also relates to a method for identifying genetic markers and/or combinations of genetic markers that are predictive of the facial characteristics, (predictive facial markers) of a person, said method comprising the steps of: a) capturing images of a group of individual faces; b) performing image analysis on facial images of said group of individual faces thereby extracting phenotypical descriptors of the faces; c) obtaining data on genetic variation from said group of individual and d) performing a genome-wide association study (GWAS) to identify said predictive facial markers.

## Description

All patent and non-patent references cited in the application are hereby incorporated by reference in their entirety.

### Field of invention

The present invention relates to a method for the generation of a facial composite from the genetic profile of a DNA-donor. The method comprises the steps of a) subjecting a biological sample to genotyping thereby generating a profile of genetic markers associated to numerical facial descriptors (NFD) for said sample, b) reverse engineering a NFD from the profile of the associated genetic variants and constructing a facial composite from the reverse engineered numerical facial descriptors (NFDs). The present invention also relates to a method for identifying genetic markers and/or combinations of genetic markers that are predictive of the facial characteristics, (predictive facial markers) of a person, said method comprising the steps of: a) capturing images of a group of individual faces; b) performing image analysis on facial images of said group of individual faces thereby extracting phenotypical descriptors of the faces; c) obtaining data on genetic variation from said group of individual and d) performing a genome-wide association study (GWAS) to identify said predictive facial markers.

### Background of the invention

Human beings differ only by up to 0.1 % of the three billion nucleotides of DNA present in the human genome. Though we are 99.9% identical in genetic sequence, it is the 0.1 % that determines our uniqueness. Our individuality is apparent from visual inspection - almost anyone can recognize that people have different facial features, heights and colors. Moreover, these features are, to some extent, heritable.

Related individuals and in particular identical twins have similar facial characteristics, suggesting that most facial characteristics are hereditary. However, only a limited number of genetic markers, predictive of the appearance of a person, are as of yet known (Han, J. et al. 2008 and Kayser, M. et al. 2008).

Some phenotypical characteristics have already been shown to be genetically inheritable. Polymorphisms have been shown to play a role in human pigmentation and height (Van Daal, A. DNA Identikit: Use of DNA Polymorphisms to predict offender appearance, from the Promega Website:
http://www.promega.com/GENETICIDPROC/ussymp18proc/oralpresentations/vanDaal.
pdf).

In genetic epidemiology, a genome-wide association study (GWA study, or GWAS) - also known as whole genome association study (WGA study) - is an examination of genetic variation across the genomes of a cohort of individuals, designed to identify genetic associations with observable traits. In human studies, this might include traits such as blood pressure, weight or occurrence of a given disease or condition.

In the last couple of years GWAS has experienced a tremendous development. Most GWAS have focused on disease-gene finding but population studies mapping the variances within and between human populations (HapMap) and recently detailed studies of the European population revealed a striking correspondence between the genetic and geographical location (Novembre, J. et al. 2008).

Studies using GWAS have previously been successfully conducted. One example is the work of an Icelandic-Dutch research collaboration, in which several Single Nucleotide Polymorphisms (SNPs) were found to be associated with human pigmentation (Sulem, P. et al. 2007, Sulem, P. et al.2008). Also, Dr Angela van Daal from the Bond University in Adelaide, Australia, has successfully associated features like skin pigmentation, eye and hair color with SNPs already suspected to be associated with these.

Despite these advances, it has not previously been possible to reconstruct an image of an individual solely from the information available in a biological sample. This is a problem in for instance crime cases, where no witnesses are available and the perpetrator only has left behind a biological trace such as skin, hair, blood or semen.

### Summary of invention

The inventors have surprisingly discovered that predictive facial markers may be discovered using Genome-Wide Association Study (GWAS) in combination with advanced image analysis. Such a method may be used to construct a facial composite from a DNA sample.

The most obvious application for the method, to predict facial appearances from DNA samples, lies within forensics. When a crime has been committed and the perpetrator has vanished - leaving only DNA traces at the crime scene - it would be of value if such a DNA trace could facilitate the apprehension of a suspect. A facial portrait would not need to qualify as legal evidence, since an apprehended suspect may be associated to the crime scene by traditional DNA profiling. Police composites based on witness descriptions are already in use and demonstrate their value in forensic science. However, witness-based police composites have some obvious limitations, namely that there has to be at least one witness and that the quality of the drawing depends on both the memory of the eyewitness and the ability to convey the memory to a sketch, possibly through a composite artist. Another possible use within forensics would be in the identification of otherwise unidentifiable corpses or body parts (in particular individuals without known relatives).

Outside forensics the method may be used to generate images of historical persons. In addition, the data set will be very educational in understanding human genetic traits under near neutral selection and could be used as a supplementary control cohort for various disease GWAS.

In order to facilitate the generation of a facial composite from a DNA sample it is first necessary to identify genetic facial markers that are predictive of the facial characteristics, (predictive facial markers) of a person.

A first aspect of the present invention thus relates to a method for identifying genetic markers that are predictive of the facial characteristics, (predictive facial markers) of a person, said method comprising the steps of:
a. capturing images of a group of individual faces;
b. performing image analysis on facial images of said group of individuals' faces thereby extracting phenotypical descriptors of the faces;
c. obtaining data on genetic variation from said group of individuals
d. performing a genome-wide association study (GWAS) to identify said predictive facial markers.

One embodiment of this aspect further comprises constructing a "face-basis" that facilitates generation of approximate facial images from a phenotypical descriptor/ numerical facial descriptor (NFDs).

Once predictive facial markers have been identified it is possible to construct a facial composite from a DNA sample.

Thus a second aspect of the invention relates to a method for generating a facial composite from a genetic profile comprising the steps of:
a. subjecting a biological sample to genotyping thereby generating a genetic profile of the genetic markers associated to the numerical facial descriptors for said sample;
b. reverse engineering a NFD from the profile of the associated genetic variants;
c. constructing a facial composite from the reverse engineered numerical facial descriptors (NFDs)

### Description of Drawings

Figure 1: Graphical outline of the research strategy.
Figure 2: AAM training from left to right: Input image, manual annotation, mesh overlay, normalized texture.

### Detailed description of the invention

### Definitions

Active Appearance Model (AAM): as used herein is intended to mean a statistical model of the variation of both the shape and the texture (the colour/grey level variation) of an object of interest, herein a face.

Facial characteristics: as used herein is intended to cover both the visual characteristics of the superficial facial characteristics and / or features such as for example the subject's faces and hair, as well as any underlying features that are only detectable using advanced image producing devices e.g. bone, cartilage, muscle structure and the like.

Dense point correspondence: as used herein is intended to mean point-to-point mapping from one facial image onto another, where each point gets the correspondent point according to its inherent property, specifically for example the points of the nose tip on different facial images are correspondent points.

"Face-basis": as used herein is intended to mean a mathematical "key" that can be used to generate a facial composite from an NFD that has been predicted from a genetic profile.

Facial composites: as used herein is intended to mean a graphical representation/reconstruction of a facial image.

Feature space: as used herein is intended to mean an abstract space where each pattern sample is represented as a point in n-dimensional space. The number of features used to describe the patterns determines its dimensionality.

Feature vectors: as used herein is intended to mean an unreduced n-dimensional vector of numerical features that represent all features of a face. Following dimension reduction the feature vector transforms into a numerical facial descriptor (NFD).

Numerical facial descriptors (NFDs) and phenotypical or facial descriptors are used interchangeably: and as used herein are intended to mean a redundancy reduced n-dimensional phenotypical numerical vector that represents the characteristic features of a face. NFDs result from dimensional reduction of feature vectors.

Genetic markers: as used herein is intended to mean a known genetic variance that may or may not associate with a particular trait. The variation may be down to the level of a Single Nucleotide Polymorphism (SNP), but may also be a larger region (of a chromosome) that is duplicated or missing (Copy Number Variation, CNV; or mini-satellites).

Genome-wide association study (GWAS): as used herein is intended to mean an examination of genetic variation across a cohort of individuals to identify genetic markers or variants that associate (correlate) with phenotypical traits (e.g. NFDs).

Haplotype: as used herein is intended to mean a set of genetic markers that are inherited together as a consequence of their chromosomal co-localization. Haplotype may refer to as few as two genetic variants or to an entire chromosome depending on the number of recombination events that have occurred between a given set of variants.

Image: as used herein is intended to mean any image captured by any Image producing device including but not limited to those described herein below and may be two-dimensional (a picture), that has a similar appearance to some subject-usually a physical object or a person, herein a face and/or skull. Images may also be three-dimensional.

Image producing device: as used herein is intended to mean any device that is capable of capturing/producing an image including but not limited to 2D cameras, 3D cameras, infrared cameras, regular cameras, scanners (e.g.: MRI, PET, CT). For more details see herein below.

Independent component analysis (ICA): as used herein is intended to mean a method for the reduction of dimensionality, specifically a computational method for separating a multivariate signal into additive sub components supposing the mutual statistical independence of the non-Gaussian source signals.

Predictive facial markers: as used herein is intended to mean any genetic marker that is predictive of facial characteristics, such as any of the facial characteristics described herein above.

Principal component analysis (PCA): as used herein is intended to mean a method for the reduction of dimensionality, specifically a mathematical procedure that transforms a number of possibly correlated variables into a smaller number of uncorrelated variables called principal components. Principal component analysis may also be referred to as Karhunen-Loève transform (KLT), the Hotelling transform or proper orthogonal decomposition (POD).

Reduction of dimensionality: as used herein is intended to mean the process of reducing the number of numerical variables under consideration, specifically a reduction in the number of descriptors (feature vectors) into NFDs that describe a face may also be referred to as extracting phenotypical descriptors.

Reverse engineering: as used herein is intended to mean the process of discovering the technological principles of a device, object or system through analysis of its structure, function and operation. Herein, specifically it refers to the reverse engineering of an NFD based on genetic variants that associate with NFDs.

Simple rectangular Haar-like features: as used herein is intended to mean the difference of the sum of pixels of areas inside the rectangle, which can be at any position and scale within the original image. This modified feature set is called 2 *rectangle feature.* Viola and Jones (2004) also defined 3 rectangle features and 4 rectangle features.

Single-nucleotide polymorphism (SNP): as used herein is intended to mean DNA sequence variation occurring when a single nucleotide in the genome (or other shared sequence) differs between members of a species or between paired chromosomes in an individual.

Sparse PCA: as used herein is intended to mean a method for the reduction of dimensionality, specifically a specialized form of PCA, Sparse PCA finds sets of sparse vectors for use as weights in the linear combinations while still explaining most of the variance present in the data

SVD singular value decomposition: as used herein is intended to mean a variant of PCA and is used for reduction of dimensionality or factorization of a rectangular real or complex matrix.

Training set: as used herein is intended to mean a set of facial images or numerical facial descriptors (NFDs) from genetically profiled individuals.

Training sample: as used herein is intended to mean a facial image or numerical facial descriptor (NFDs) from a genetically profiled individual.

### Embodiments

In a main embodiment the invention relates to a method for generating a facial composite from a genetic profile comprising the steps of:
a. subjecting a biological sample to genotyping thereby generating a profile of the genetic markers associated to the numerical facial descriptors (NFD) for said sample;
b. reverse engineering a NFD from the profile of the associated genetic variants.
c. constructing a facial composite from the reverse engineered numerical facial descriptors (NFDs)

Another main embodiment relates to a method for identifying genetic markers that are predictive of the facial characteristics, (predictive facial markers) of a person, said method comprising the steps of:
a. capturing images of a group of individual faces;
b. performing image analysis on facial images of said group of individual's faces thereby extracting phenotypical descriptors of the faces;
c. obtaining data on genetic variation from said group of individuals
d. performing a genome-wide association study (GWAS) to identify said predictive facial markers.

### The phenotypical descriptor

As described herein above and in more detail below, the inventors use a Genome-Wide Association Study (GWAS) in combination with advanced image analysis. The statistical power needed for a GWAS to identify genetic markers depends mainly on the following four factors:
- The phenotypical descriptor must correspond well to genetic components.
- The genetic markers must occur with a reasonable high frequency in the cohort investigated.
- The genetic marker(s) should preferably have high penetrance.
- A well stratified population where phenotypical traits are not confounded with origin of decent.

The first factor will be met by identifying appropriate descriptors, as described here: Image processing tools already exist for extracting facial descriptors from images (described herein below). Adjusting parameters in the dimension reduction process of the image data (feature vectors) may ensure maximal correspondence to the underlying genetic variations. This can be done by selecting the phenotypical descriptors (eg. PCA components) that minimize separation between genetically related individuals (eg. brothers) and maximize separation between distantly related persons. In this way the phenotypical descriptor is optimized for correspondence to the genetic components.

Similarly, and to meet the second factor, the phenotypical descriptors may be selected to describe frequent, yet discriminative, features of the face.

Third, disease association studies tend to show that genetic variants with high penetrance are rare. This can best be explained for genetic variants that are subjected to negative selection, as is the case for most disease causing variants with strong penetrance. For facial characteristics, however, most variants are less likely to cause strong negative selection. In support of this, some facial features such as cheek dimples, cleft chin, free or attached earlobes, face freckles and widow's peak are common and follow Mendelian inheritance (i.e. 100% penetrance). Other features, like eye color, hair color and skin pigmentation, are influenced by few genetic loci with high penetrance.

Finally, as GWAS is based on correlations between genetic markers and phenotypical traits it is important that correlations between the phenotypes and the origin of decent are controlled or avoided, as population specific markers are likely to result in a masking of true causative loci. This problem is best avoided by stratified sampling from a population homogeneous of decent.

### Image capture/phenotypical detection

The present invention relates to methods for identifying predictive facial markers, wherein the first step comprises phenotypical detection by capturing images of a group of individual faces including both surface and non-surface anatomical features.

3D-shape models of the human face have previously been used to discriminate between patients with different genetically related facial dysmorphologies, including Noonan syndromes and 22q11 deletion syndrome (Hammond, P. et al 2004, Hammond, P. et al 2005).

The images of said faces may be captured using any image producing device selected from the group consisting of 2D cameras, 3D cameras, infrared cameras, regular cameras or any combination of those, such as a 2D camera and a 3D camera, for example a 2D camera and a regular camera, such as a 2D camera and an infrared camera, for example a 3D camera and a regular camera, such as a 3D camera and an infrared camera, for example a regular camera and an infrared camera.

Recently, techniques have been developed to enable computed tomography (CT), magnetic resonance imaging (MRI), PET (positron emission tomography) and ultrasound scanning software to produce 3D images. Traditionally CT and MRI scans produced 2D static output on film. To produce 3D images, many scans are made, which are then combined by computers to produce a 3D model. 3D ultrasounds are produced using a somewhat similar technique.

Other types of image producing systems are also contemplated to be within the scope of the present invention. These includes image producing systems that are capable of producing an image of both surface and non-surface anatomical features such as but not limited to X-ray, ultrasound, such as ultrasonography, computer-transformed images, IR, terahertz, electron microscopy, radiography, magnetic resonance imaging (MRI), Photoacoustic imaging, thermography, optical imaging.

Any useful type of tomography such as but not limited to optical coherence tomography, computed tomography or Computed Axial Tomography (CAT), linear tomography, poly tomography, zonography and Electrical impedance tomography may also be used.

Gamma cameras including 2D planar images that may be acquired of the face or multiple time-capture images can be combined into a dynamic sequence of a physiologic process over time and the 3D tomographic technique known as SPECT that uses gamma camera data from many projections and can be reconstructed in different planes. A dual detector head gamma camera combined with a CT scanner, which provides localization of functional SPECT data, is termed a SPECT/CT camera and is also comprised within the scope of the present invention.

Any of these methods and/or devices may be used alone or in combination with any other image producing device and/or system.

Thus phenotypical detection by image capture of a group of individual faces may be done using any of the methods and/or devices and/or combinations of these, described herein above, for example selected from the group consisting of 2D cameras, 3D cameras, infrared cameras, regular cameras, scanners (e.g.: MRI, PET, CT), X-ray, ultrasound, such as ultrasonography, computer-transformed images, IR, terahertz, electron microscopy, radiography, magnetic resonance imaging (MRI), Photoacoustic imaging, thermography, optical imaging, optical coherence tomography, computed tomography or Computed Axial Tomography (CAT), linear tomography, poly tomography, zonography and Electrical impedance tomography, gamma cameras and SPECT.

### Facial Image Analysis and Feature Extraction

The recognition and identification of faces and feature extraction from face images is a very active research area, and there is a wide range of applications. Face detection systems essentially operate by scanning an image for regions having attributes which would indicate that a region contains the face of a person. These systems operate by comparing some type of training images depicting people's faces (or representations thereof) to an image or representation of a person's face extracted from an input image. Furthermore, face detection is the first step towards automated face recognition

In Viola et al. (2004) simple Haar-like features are extracted; face/non-face classification is done by using a cascade of successively more complex classifiers which are trained by using the (discrete) AdaBoost learning algorithm. This resulted in the first real-time frontal face detection system which runs at about 14 frame per second for a 320*240 image

Any method known in the art may be used for the recognition and identification of faces and feature extraction from face images such as but not limited to real-time surveillance, camera auto-focus, biometry, image-based diagnostics and the Viola-Jones face detector ⁽Viola and Jones (2004).

For the present invention the method for the feature extraction from face images should be able to do the following:
- Describe a set of faces using features vectors
- Reduce the dimensionally, so that each face is described by a small subset of parameters/components
- Synthesize new face representations/images given such arbitrary face-parameter vectors/ numerical facial descriptors (NFDs)

The Viola and Jones (2004) method combines weak classifiers based on simple binary features which can be computed extremely fast. Simple rectangular Haar-like features are extracted; face and non-face classification is done using a cascade of successively more complex classifiers which discards non-face regions and only sends face-like candidates to the next layer's classifier. Thus it employs a "coarse-to-fine" method. Each layer's classifier is trained by the AdaBoost learning algorithm. Adaboost is a boosting learning algorithm which can fuse many weak classifiers into a single more powerful classifier. In a preferred embodiment the Viola-Jones face detector is used for the recognition and identification of faces and feature extraction from face images.

The cascade face detector finds the location of a human face in an input image and provides a good starting point for the subsequent AAM search.

### Active Appearance Models (AAM)

An active appearance model (AAM) is a computer vision algorithm for matching a statistical model of object shape and appearance to a new image. They are built during a training phase. A set of images together with coordinates of landmarks, that appear in all of the images is provided by the training supervisor.

Active Appearance Models or Facial Feature Interpretation is a powerful tool to describe deformable object images. It demonstrates that a small number of 2D statistical models are sufficient to capture the shape and appearance of a face from any viewpoint. The Active Appearance Model may use any variant of principal component analysis (PCA, see herein below) on the linear subspaces to model both geometry (3D location) and texture (color) of the object in interest, herein the image of a face. AAM was originally described as a method working with 2D images, but now the method has been extended to 3D data - in particular 3D surface data. The model is a so-called learning-based method, where an input-data set is used to parameterize the model. Given a collection of training images for a certain object class where the feature points have been manually marked, shape and texture can be represented for example by applying PCA to the sample shape and texture distributions as: x = ^ + Psc (1) and g = <∼>g+Pgc (2) where x is the mean shape, g is the mean texture and Ps , Pg are matrices describing the respective shape and texture variations learned from the training sets. The parameters, c are used to control the shape and texture change.

The AAM search precisely marks the major facial features, such as mouth, eyes, nose and so on.

In a preferred embodiment of the invention the image analysis comprises using an AAM.

Thus, in a very preferred embodiment the image analysis comprises using an Active Appearance Model (AAM) to extract phenotypical descriptors of said group of faces (a training set), the method comprising the steps of:
a. generating a dense point correspondence over the training set;
b. aligning the individual dense point correspondence in said training set;
c. generating feature vectors by sampling geometry (3D location) and texture (color) according to the dense point correspondence;
d. reducing the dimensionality, so each face/training sample is described by a small and independent subset of components or numerical facial descriptors (NFDs); wherein the reduction in dimensionality additionally generates a "face-basis" that facilitates generation of approximate facial images from the NFDs.

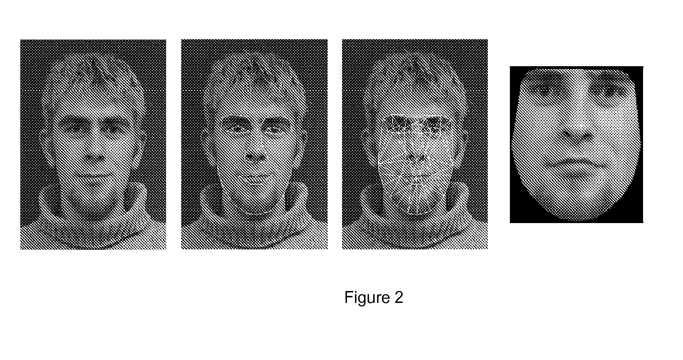

In the first step, facial characteristics and/or features are identified in each training sample (face). This may be done either manually by identifying landmarks such as but not limited to the tip of the nose, the chin, the ears, and so forth, or automatically using different algorithms. An example of a manually annotated face image can be seen in Figure 2. The basis of the identification of landmarks is that it may result in a point-wise correspondence over the training set.

Thus in one particular embodiment the dense point correspondence comprises facial characteristics and/or features identified in each training sample (face).

In another embodiment the facial characteristics and/or features are aligned across the training set by identifying landmarks such as the tip of the nose, the chin, the ears, and so forth.

Secondly, the training set may be aligned. The dense point correspondence across the training set may be aligned using a method selected from the group consisting of generalized Procrustes analysis and any other useful method known in the art. In a preferred embodiment the dense point correspondence across the training set mat be aligned using generalized Procrustes analysis.

From these aligned shapes feature vectors may be extracted. In most approaches the feature vectors consists of a mix of the spatial locations of the points defining the shapes and the color values defining the texture.

However, a feature vector consisting of hundreds of thousands of values, with huge redundancy, now describes each face. Thus it is essential to reduce the dimensionality so that each face is described by fewer parameters/components. Each face may thus be described by less than 1000 parameters/components, for example by less than 900 parameters/components, such as by less than 800 parameters/components, for example by less than 700 parameters/components, for example by less than 600 parameters/components, such as by less than 500 parameters/components, for example by less than 400 parameters/components, such as by less than 300 parameters/components, for example by less than 250 parameters/components, such as by less than 200 parameters/components, for example by less than 150 parameters/components, such as by less than 100 parameters/components, for example by less than 50 parameters/components, such as by less than 25 parameters/components. In a preferred embodiment each face is described by less than 50 parameters/components.

Thus in a preferred embodiment of the invention, each training sample (facial image) is described by less than 50 components following the reduction in dimensionality.

Any dimensionality-reduction techniques may be used to reduce the dimensionality such as but not limited to any mathematical procedure that transforms a number of possibly correlated variables into a smaller number of uncorrelated variables called principal components including but not limited to Principal Component Analysis (PCA), Independent Component Analysis (ICA), and Sparse-PCA. However, while PCA creates a basis based on a maximization of the explained variance, an alternative approach may be more suitable for the goal of this study. Such alternative, may maximize the distance between family-wise unrelated individuals and minimize the distance between family-wise related individuals, or select components that describe common features.

Without being bound by theory, it is contemplated that genetically related individuals have similar facial characteristics and thus that the NFDs extracted from images of people that are genetically related should lie close in *feature space,* and the reverse for people that are not related, a reduced sub-space based on these objectives could be computed. Thus, in some embodiments similar numerical facial descriptors (NFDs) are extracted for genetically related individuals and distinct NFD's are extracted for unrelated individuals.

Thus, in a preferred embodiment the dimensionality is reduced using a technique selected from the group consisting of: principal component analysis (PCA), independent component analysis (ICA), adaptive PCA and sparse PCA or derivates thereof.

After dimensionality reduction, a low-dimensional feature vector - the numerical facial descriptor (NFD), will describe each face in the training set.

### Genetic marker

A predictive facial genetic marker within the scope of the present invention may be any gene or DNA sequence or absence of such, with a known location on a chromosome and associated with a particular facial phenotype.

It may be a variation, which may arise due to mutation or alteration in the genomic loci that can be observed. A genetic marker may be a short DNA sequence, such as a single base-pair change (single nucleotide polymorphism, SNP), or a longer one, like copy number variation (CNV) or variable number tandem repeats such as mini-satellites or microsatellites. The genetic variation may also be epigenetics a type of variation that arises from chemical tags that attach to DNA and affect how it gets read.

In a particularly preferred embodiment the genetic marker is a SNP/genetic variant.

A genetic variant may be any genetic polymorphism observed in the cohort studied, including but not limited to single nucleotide polymorphisms (SNP), copy number variation (where a larger region is duplicated or missing), DNA inversions, any type of epigenetic variations. Genetic variations may be investigated at the level of haplotypes where sets of genetic variations are co-inherited. Associations between a phenotypical trait and to a genetic variant may not necessarily mean that the variant is causative for the trait.

Thus the genetic marker may be a genetic variation selected from the group consisting of single nucleotide polymorphism (SNP), copy number variation (CNV), epigenetics and DNA inversions.

### Genome-wide Association Studies

Most genetic variations are associated with the geographical and historical populations in which the mutations first arose. This ability of SNPs to tag surrounding blocks of ancient DNA (haplotypes) underlies the rationale for GWAS.

After the generation of the Numerical Facial Descriptors (NFDs), predictive genetic facial markers may be identified. This may be pursued by associations between these and genetic variations collected genome wide; a genome-wide association study (GWAS).

The statistical power needed for a GWAS to identify predictive facial genetic markers depends mainly on the following four factors:
- How well the phenotypical descriptor correspond to the genetic components.
- How frequent the genetic markers occur in the investigated cohort
- The penetrance of the genetic marker(s)
- A well stratified population where phenotypical traits are not confounded with origin of decent

Statistical power is very important in GWAS. Even with a cohort size of 1,000 persons a GWAS will be limited in detecting associations to only the subset of genetic variants that have relatively high penetrance and are sufficiently represented in the cohort. Association of facial characteristics may benefit from several advantages over the classical case-control GWAS. First, variants in the facial characteristics may experience close to neutral selection, and consequently variants with high penetrance may be frequent. Second, in contrast to most case-control categorization, the NFD is a continuous descriptor (quantitative trait), resulting in a significant increase of statistical power in the association analysis.

Any method known to a person skilled in the art may be used for the GWAS. This may for example be any method that may be used in the identification of genetic markers on a genome-wide level including but not limited to genome-wide arrays or any form of DNA sequencing. DNA sequencing methods are well known in the art and include but are not limited to for example chemical sequencing, Chain-termination methods, Dye-terminator sequencing, In vitro clonal amplification, Parallelized sequencing, Sequencing by ligation, nano-pore DNA sequencing, 454 sequencing, Microfluidic Sanger sequencing and Sequencing by hybridization. Any such method is envisioned to be comprised within the scope of the present invention¤¤. In preferred embodiments any sequencing method may be used.

Genome-wide microarray arrays for GWAS, include but are not limited to Affymetrix Genome-Wide Human SNP 6.0 arrays and , Affymetrix Genome-Wide Human SNP 5.0 arrays, lllumina HD BeadChip, NimbleGen CGH Microarrays, Agilent GCH. In a particularly useful embodiment Affymetrix Genome-Wide Human SNP 6.0 array is used.

The Affymetrix Genome-Wide Human SNP 6.0 array have been developed based on results generated by the international *Haplotype Mapping* (HapMap (Thorisson G.A. et al., 2005)) project and measure more than 1.8 million genetic markers - 900.000 SNPs and 900.000 copy number variations (CNVs) in the human genome. Genetic data is highly redundant, since many genetic variants are coupled together in haplotypes.

Any method that analyses haplotypes rather than individual SNPs and CNVs are particularly preferred, since this decreases the signal-to-noise ratio and reduces the number of variants to be analyzed, which consequently reduces the number of hypothesizes to be tested.

Here a genetic variant means any genetic polymorphism observed in the cohort studied, including but not limited to Single Nucleotide Polymorphisms (SNP), Copy Number Variation (CNV), Chromosomal inversions, any type of epigenetic variations. Genetic variations may be investigated at the level of haplotypes where sets of genetic variations are co-inherited. Associations between a phenotypical trait and a genetic variant may not necessarily mean that the variant is causative for the trait.

Several methods for identifying a genetic variant will be know to a person skilled in the art. Any such method may be employed to determine if a genetic variation associate to the phenotypical observation. Both discreet phenotypical observation such as eye color as well as continuous observations such as height may associate to a genetic variation, being a single SNP or a set of different genetic variations.

Following the identification of genetic variants that correlate with the NFD, an probabilistic NFD predictor will be trained and benchmarked on a distinct subset of the cohort (cross-validation/leave-one-out testing).

Thus in one particular embodiment the genome-wide association study (GWAS) comprises the steps of:
a. analyzing the haplotype of the genetic profiles;
b. identifying genetic variants that through out the sample cohort correlate/associate with the numerical facial descriptors (NFDs), thereby identifying a genetic marker associating to a phenotypical feature.

### Haplotype analysis

Any haplotype analysis known in the art may be used. WO 03/048372 and US 7,107,155 describe methods for correlating/associating genetic variations with traits. Any of the methods described herein may be used for the present and both WO 03/048372 and US 7,107,155 are hereby incorporated by reference.

In one preferred embodiment wherein the haplotype analysis comprises performing an iterative analytical process on a plurality of genetic variations for candidate marker combinations; the iterative analytical process comprising the acts of:
a. selecting one candidate combination of genetic variations from the pool of all candidate combinations of genetic variations;
b. reading haplotype data associated with the candidate combination for a plurality of individuals;
c. correlating the haplotype data of the plurality of individuals according to facial characteristics (as scored by NFD);
d. performing a statistical analysis on the haplotype data to obtain a statistical measurement associated with the candidate combination;
e. repeating the acts of selecting (a), reading (b), correlating (c), and performing statistical analysis(d) as for additional combinations of genetic variations in order to identify one or more optimal combinations from the pool of all candidate combinations of genetic variations.

### Generating a facial composite from NFDs and identified genetic variants

From the methods described herein above a facial composite may be constructed from a NFDs predicted from associated genetic variants. Hence, given an NFD an approximate set of feature vectors can be constructed by a reverse dimension reduction. In the case where PCA was used for dimension reduction/conversion of the original feature vectors to NDFs (eigen vector) the rotation matrix, which is a derivative of the dimension reduction is used to reconstruct approximate feature vectors from the NDFs.

The set of reconstructed feature vectors then facilitates construction of a facial composite through an AAM derived "face basis".

Hence, a "face-basis" may be used to construct the facial composite (sketch) from a NFD, predicted from a genetic profile of yet unseen subject.

### Generating a facial composite from a genetic profile

In order to facilitate the generation of a facial composite from a DNA sample it is firstly necessary to identify genetic facial markers that are predictive of the facial characteristics of a person (predictive facial markers), described herein below.

Another embodiment of the invention relates to a method for generating a facial composite from a genetic profile comprising the steps of:
1. subjecting a biological sample to genotyping thereby generating a profile of the genetic markers associated to the numerical facial descriptors (NFD) for said sample;
2. reverse engineer a NFD from the profile of the associated genetic variants.
3. constructing a facial composite from the reverse engineered numerical facial descriptors (NFDs)

In a specific embodiment the biological sample is collected from the group consisting of blood, saliva, hair, bone, semen or flesh.

In another specific embodiment the genetic profile is correlated/associated with the facial descriptor/numerical facial descriptors (NFDs).

A given genetic variation or combination of variations scored through a cohort of individuals, are associated to the component of a facial descriptor scored through the same cohort of individuals. For these; regression, correlation, odd ratio and statistical significance may be calculated, the methods for which are known for a skilled person.

In another specific embodiment the facial composite is generated as described herein above.

### Detailed description of the drawings

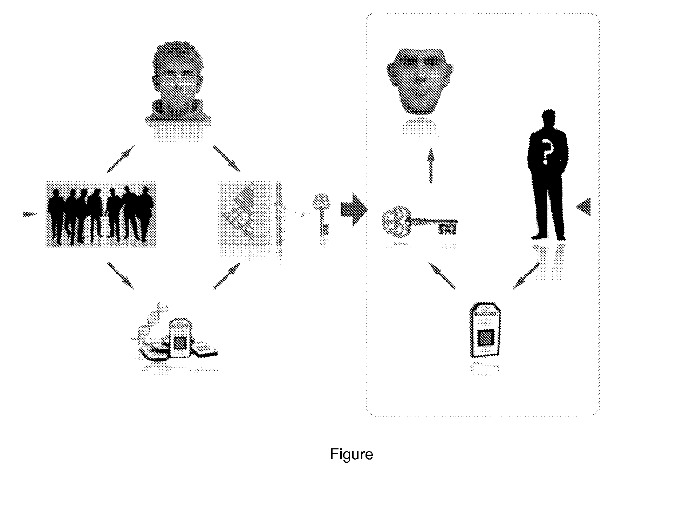

Figure 1: Graphical outline of the research strategy. The first step corresponds to *image registration* where anatomical or pseudo-anatomical features are identified in each training sample.
Figure 2: AAM training from left to right: Input image, manual annotation, mesh overlay, normalized texture. Example of a manually annotated face image, The basis of the registration is that it should result in a point-wise correspondence over the training set.
In the second step, the training set is aligned using a so-called generalized Procrustes analysis and from these aligned shapes feature vectors can be extracted.

### Examples

### Cohort and Data

1,000 ethnic Danish male subjects will be used. They should be without facial hair and within the age range of 25 to 30 years. All subjects will be photographed using a 3D camera and their genetic profiles will be determined by hybridizing blood-extracted DNA to an Affymetrix Genome-Wide Human SNP 6.0 array at the DNA-MicroArray Core (D-MAC) facility.

### Research Strategy

Image analysis and GWAS will be combined. The overall strategy is shown schematically in Figure 2.

### Items

One aspect of the invention relates to a method for generating a facial composite from a genetic profile comprising the steps of:
a. subjecting a biological sample to genotyping thereby generating a profile of the genetic markers associated to the numerical facial descriptors (NFD) for said sample;
b. reverse engineer a NFD from the profile of the associated genetic variants.
c. constructing a facial composite from the reverse engineered numerical facial descriptors (NFDs)

In another embodiment said biological sample is collected from the group consisting of blood, saliva, hair, bone, semen and flesh.

In yet another embodiment said genetic profile is correlated with the facial descriptor /numerical facial descriptors (NFDs).

In another embodiment said facial composite is generated.

Another aspect of the invention relates to a method for identifying genetic markers and/or combinations of genetic markers that are predictive of the facial characteristics, (predictive facial markers) of a person, said method comprising the steps of:
a. capturing images of a group of individual faces;
b. performing image analysis on facial images of said group of individual faces thereby extracting phenotypical descriptors of the faces;
c. obtaining data on genetic variation from said group of individuals
d. performing a genome-wide association study (GWAS) to identify said predictive facial markers.

In another embodiment the method further comprises the generation of a "face-basis" that facilitates generation of approximate facial images from phenotypical descriptors/ NFDs

In another embodiment the images of said faces are captured using a device selected from the group consisting of 2D cameras, 3D cameras, infrared cameras, regular cameras, scanners (e.g.: MRI, PET, CT), X-ray, ultrasound, such as ultrasonography, computer-transformed images, IR, terahertz, electron microscopy, radiography, magnetic resonance imaging (MRI), Photoacoustic imaging, thermography, optical imaging, optical coherence tomography, computed tomography or Computed Axial Tomography (CAT), linear tomography, poly tomography, zonography and Electrical impedance tomography, gamma cameras and SPECT.

In yet another embodiment the image analysis comprises using an Active Appearance Model (AAM) to extract phenotypical descriptors of said group of faces (a training set), the method comprising the steps of:
a. generating a dense point correspondence over the training set;
b. aligning the individual dense point correspondence in said training set;
c. generating feature vectors by sampling geometry (3D location) and texture (color) according to the dense point correspondence;
d. reducing the dimensionality, so each face/training sample is described by a small and independent subset of components or numerical facial descriptors (NFDs); wherein the reduction in dimensionality additionally generates a "face-basis" that facilitates generation of approximate facial images from the NFDs.

In still another embodiment the dense point correspondence comprises aligning facial characteristics and/or features identified in each training sample (face).

In another embodiment the dense point correspondence across the training set is aligned using generalized Procrustes analysis.

In yet another embodiment the facial characteristics and/or features are aligned across the training set by identifying landmarks such as the tip of the nose, the chin, the ears, and so forth.

In still another embodiment the dimensionality is reduced using a technique selected from the group consisting of: principal component analysis (PCA), independent component analysis (ICA), adaptive PCA and sparse PCA.

In another embodiment similar NFDs are extracted for genetically related individuals and distinct NFD's are extracted for unrelated individuals.

In yet another embodiment each training sample (facial image) is described by less than 50 components following the reduction in dimensionality.

In still another embodiment the genetic marker is a genetic variation selected from the group consisting of single Nucleotide Polymorphisms (SNP), Copy Number Variation (CNV), Chromosomal inversions, any type of epigenetic variations.

In another embodiment the data on genetic variation of said group of individuals is obtained by:
a. obtaining a biological sample from each subject;
b. subjecting the biological samples to genotyping, generating genetic profiles for said subjects.

In yet another embodiment the genome-wide association study (GWAS) comprises the steps of:
a. analyzing the haplotype of the genetic profiles;
b. identifying genetic variants that through out the sample cohort correlate/ associate with the numerical facial descriptors (NFDs) of claim 8, thereby identifying a genetic marker and/or combinations of genetic markers associating to a phenotypical feature.

In still another embodiment the haplotype analysis comprises performing an iterative analytical process on a plurality of genetic variations for candidate marker combinations; the iterative analytical process comprising the acts of:
a. selecting one candidate combination of genetic variations from the pool of all candidate combinations of genetic variations;
b. reading haplotype data associated with the candidate combination for a plurality of individuals;
c. correlating the haplotype data of the plurality of individuals according to facial characteristics (as scored by NFD);
d. performing a statistical analysis on the haplotype data to obtain a statistical measurement associated with the candidate combination;
e. repeating the acts of selecting (a), reading (b), correlating (c), and performing statistical analysis(d) as for additional combinations of genetic variations in order to identify one or more optimal combinations from the pool of all candidate combinations of genetic variations.

### References

Han, J. et al. A Genome-Wide Association Study Identifies Novel Alleles Associated with Hair Color and Skin Pigmentation. PLoS Genetics 4, (2008).
Kayser, M. et al. Three Genome-wide Association Studies and a Linkage Analysis Identify HERC2 as a Human Iris Color Gene. The American Journal of Human Genetics 82, 411-423 (2008).
Sulem, P. et al. Two newly identified genetic determinants of pigmentation in Europeans. Nature Genetics 40, 835 (2008).
Sulem, P. et al. Genetic determinants of hair, eye and skin pigmentation in Europeans. Nature Genetics 39, 1443 (2007).
Viola, P. & Jones, M. J. Robust Real-Time Face Detection. International Journal of Computer Vision 57, 137-154 (2004).
Hammond, P. et al. 3D Analysis of Facial Morphology. American Journal of Medical Genetics 126, 339-348 (2004).
Hammond, P. et al. Discriminating Power of Localized Three-Dimensional Facial Morphology. The American Journal of Human Genetics 77, 999-1010 (2005).
Thorisson, G. A., Smith, A. V., Krishnan, L. & Stein, L. D. The International HapMap Project Web site. Genome Research 15(11), 1592-1593 (2005).
Novembre, J. et al. Genes mirror geography within Europe. Nature (2008).

## Claims

1. A method for generating a facial composite from a genetic profile comprising the steps of:
a. subjecting a biological sample to genotyping thereby generating a profile of the genetic markers associated to the numerical facial descriptors (NFD) for said sample;
b. reverse engineer a NFD from the profile of the associated genetic variants.
c. constructing a facial composite from the reverse engineered numerical facial descriptors (NFDs)

2. The method of claim 1, wherein said biological sample is collected from the group consisting of blood, saliva, hair, bone, semen and flesh.

3. The method of claim 1, wherein said genetic profile is correlated with the facial descriptor /numerical facial descriptors (NFDs).

4. A method for identifying genetic markers and/or combinations of genetic markers that are predictive of the facial characteristics, (predictive facial markers) of a person, said method comprising the steps of:
a. capturing images of a group of individual faces;
b. performing image analysis on facial images of said group of individual faces thereby extracting phenotypical descriptors of the faces;
c. obtaining data on genetic variation from said group of individuals
d. performing a genome-wide association study (GWAS) to identify said predictive facial markers.

5. The method of claim 4 further comprising the generation of a "face-basis" that facilitates generation of approximate facial images from phenotypical descriptors/ NFDs

6. The method of claim 4, wherein the images of said faces are captured using a device selected from the group consisting of 2D cameras, 3D cameras, infrared cameras, regular cameras, scanners (e.g.: MRI, PET, CT), X-ray, ultrasound, such as ultrasonography, computer-transformed images, IR, terahertz, electron microscopy, radiography, magnetic resonance imaging (MRI), Photoacoustic imaging, thermography, optical imaging, optical coherence tomography, computed tomography or Computed Axial Tomography (CAT), linear tomography, poly tomography, zonography and Electrical impedance tomography, gamma cameras and SPECT.

7. The method of claim 4, wherein the image analysis comprises using an Active Appearance Model (AAM) to extract phenotypical descriptors of said group of faces (a training set), the method comprising the steps of:
a. generating a dense point correspondence over the training set;
b. aligning the individual dense point correspondence in said training set;
c. generating feature vectors by sampling geometry (3D location) and texture (color) according to the dense point correspondence;
d. reducing the dimensionality, so each face/training sample is described by a small and independent subset of components or numerical facial descriptors (NFDs); wherein the reduction in dimensionality additionally generates a "face-basis" that facilitates generation of approximate facial images from the NFDs.

8. The method of any of claim 7, wherein the dense point correspondence comprises aligning facial characteristics and/or features identified in each training sample (face).

9. The method of claim 7, wherein the dense point correspondence across the training set is aligned using generalized Procrustes analysis.

10. The method of claim 7, wherein the facial characteristics and/or features are aligned across the training set by identifying landmarks such as the tip of the nose, the chin, the ears, and so forth.

11. The method of claim 7, wherein the dimensionality is reduced using a technique selected from the group consisting of: principal component analysis (PCA), independent component analysis (ICA), adaptive PCA and sparse PCA.

12. The method of claim 4, wherein the genetic marker is a genetic variation selected from the group consisting of single Nucleotide Polymorphisms (SNP), Copy Number Variation (CNV), Chromosomal inversions, any type of epigenetic variations.

13. The method of claim 4, wherein the data on genetic variation of said group of individuals is obtained by:
a. obtaining a biological sample from each subject;
b. subjecting the biological samples to genotyping, generating genetic profiles for said subjects.

14. The method of claim 4 and 14, wherein the genome-wide association study (GWAS) comprises the steps of:
a. analyzing the haplotype of the genetic profiles;
b. identifying genetic variants that through out the sample cohort correlate/ associate with the numerical facial descriptors (NFDs) of claim 8, thereby identifying a genetic marker and/or combinations of genetic markers associating to a phenotypical feature.

15. The method of claim 14, wherein the haplotype analysis comprises performing an iterative analytical process on a plurality of genetic variations for candidate marker combinations; the iterative analytical process comprising the acts of:
a. selecting one candidate combination of genetic variations from the pool of all candidate combinations of genetic variations;
b. reading haplotype data associated with the candidate combination for a plurality of individuals;
c. correlating the haplotype data of the plurality of individuals according to facial characteristics (as scored by NFD);
d. performing a statistical analysis on the haplotype data to obtain a statistical measurement associated with the candidate combination;
e. repeating the acts of selecting (a), reading (b), correlating (c), and performing statistical analysis(d) as for additional combinations of genetic variations in order to identify one or more optimal combinations from the pool of all candidate combinations of genetic variations.
